# EUROPÄISCHE PATENTSCHRIFT

(11) **EP 1 357 950 B1**
(45) Veröffentlichungstag und Bekanntmachung des Hinweises auf die Patenterteilung: **23.06.2004**
(21) Anmeldenummer: 02704691.1
(22) Anmeldetag: 07.02.2002
(51) Int. Cl.: A61L 15/18, A61L 15/24, A61L 15/26, A61L 15/46, A61L 9/014

(54) **GERUCHSADSORPTIONSMITTEL IN HYGIENEARTIKELN**
ODOUR ADSORPTION AGENT IN HYGIENE ARTICLES
AGENT D'ADSORPTION D'ODEURS DANS DES ARTICLES D'HYGIENE

(30) Priorität: 10.02.2001 DE 10106139
(43) Veröffentlichungstag der Anmeldung: 05.11.2003
(73) Patentinhaber: Paul Hartmann Aktiengesellschaft, 89522 Heidenheim (DE)
(72) Erfinder: MANGOLD, Rainer, 89542 Herbrechtingen (DE); HERMANN, Klaus, 89537 Giengen (DE)
(74) Vertreter: Langöhrig, Angelika Beate
(86) Internationale Anmeldenummer: PCT/EP2002/001279
(87) Internationale Veröffentlichungsnummer: WO 2002/064176

(56) Entgegenhaltungen:
- EP-A- 0 392 528
- EP-A- 0 882 485
- EP-A- 1 034 800
- US-A- 4 525 410
- US-A- 5 498 478

## Beschreibung

Die vorliegende Erfindung betrifft ein Geruchsadsorptionsmittel, welches insbesondere auf dem Gebiet der Hygieneartikel, wie Windeln, Damenbinden, Tampons sowie weiterer Inkontinenzartikel Einsatz finden soll. Das Geruchsadsorptionsmittel soll dabei ein breites Spektrum übelriechender Gerüche binden können.

Hygieneartikel werden vielfach nicht nur daraufhin ausgelegt, daß sie ein möglichst gutes Absorptionsvermögen für Körperflüssigkeiten, wie Blut, Menstruationsflüssigkeit und Urin aufweisen sollen, sondern darüber hinaus auch hinsichtlich ihrer hygienischen Eigenschaften sowie ihres Tragekomforts. Wegwerfbare Produkte wie Windeln und Slipeinlagen umfassen in der Regel eine flüssigkeitsdurchlässige Oberschicht, einen absorbierenden Kern und eine flüssigkeitsundurchlässige Unterschicht. Hygieneartikel gibt es in den verschiedensten Größen, Formen und Dicken.

Ein besonderer Aspekt derartiger Hygieneprodukte ist die Kontrolle von auftretenden Gerüchen. Viele Körperflüssigkeiten haben die unangenehme Eigenschaft, einen Geruch abzusondern oder in Verbindung mit Luft oder Bakterien für einen längeren Zeitraum einen derartigen Geruch auszubilden.

Als Geruchsadsorbentien sind im Stand der Technik verschiedenste Stoffe bekannt. Insbesondere werden Zeolithe hierzu eingesetzt. Zeolithe werden in der Regel in Form von kleinen Partikeln eingesetzt, so daß das Zeolithmaterial in Pulverform vorliegt. Ein derartiges Zeolithpulver besitzt den Nachteil, daß es nur schwierig im industriellen Rahmen zu handhaben ist. Zeolithpulver neigen dazu, zu stauben und besitzen darüber hinaus die Eigenschaft, sich von der absorbierenden Struktur des Hygieneartikels, in dem sie enthalten sind, zu lösen bzw. sich mit dieser zu entmischen. Dies tritt insbesondere bei den hohen Bearbeitungsgeschwindigkeiten, die auf modernen Maschinen beispielsweise zur Herstellung von Windeln herrschen, auf.

Es ist daher aus dem Stand der Technik bekannt, Zeolithe mit amorpher Kieselsäure zu Agglomeraten zu verbinden.

Darüber hinaus ist im Stand der Technik offenbart, beispielsweise in der EP 0 515 477 B1, Zeolithe über chemische Bindungen an das Absorptionsmittel, nämlich ein superabsorbierendes Polymer, zu binden. Durch diese Bindung werden jedoch die SAP-Eigenschaften, und damit die Absorptionseigenschaften, für Körperflüssigkeiten verändert. Darüber hinaus werden auch die Eigenschaften des Zeoliths verändert, so daß auch die Geruchsadsorptionseigenschaften sinken. Die Bindung erfolgt hierbei in Form einer kohäsiven Mischung unter Zugabe eines Bindemittels, insbesondere Hydroxypropylzellulose.

Ein weiterer Nachteil der Bindung des Zeoliths an das SAP ist die Notwendigkeit, daß das Zeolith nur zusammen mit dem SAP zudosiert werden kann, wobei jedoch die Verankerung des Zeoliths am SAP schwach ist. Wird die Bindung zwischen Zeolith und SAP aufgehoben, treten erneut die bekannten Staubprobleme auf. Es ist weiterhin beispielsweise aus der PCT/US 99/02760 bekannt, Zeolithe an eine Polymermatrix einzubinden, wobei die Matrix ein Polymer funktionaler Gruppen aufweist, die mit dem Zeolith reagieren und dieses dadurch an sich binden.

Schließlich ist es aus DE-OS 21 64 262 bekannt, Adsorbentien an sogenannte "Bikomponentenfasern" zu binden. Diese Fasern sind zum einen in der Herstellung aufwendig, zum anderen lassen sie sich nur schlecht in die absorbierende Struktur eines Hygieneartikels eingliedern.

Es ist Aufgabe der Erfindung, ein Geruchsadsorptionsmittel sowie einen Hygieneartikel und ein Verfahren zur Herstellung eines Geruchsadsorptionsmittels bereitzustellen, das einfach im industriellen Maßstab zu handhaben ist, eine gute Geruchsadsorptionsfähigkeit aufweist und besonders gut für den Einsatz in Hygieneartikeln geeignet ist.

Die Erfindung löst diese Aufgabe durch die Bereitstellung eines Geruchsadsorptionsmittels umfassend ein thermoplastisches Granulat sowie über Schmelzbindungen an das thermoplastische Granulat gebundene Zeolithe. Die Zeolithe werden hierbei auf das thermoplastische Granulat aufgeschmolzen. Eine derartige Materialkombination eignet sich besonders gut, um den in der Hygieneindustrie üblicherweise eingesetzten SAPs (superabsorbent polymers) beigemischt zu werden. Es sind insbesondere keine weiteren Dosiereinrichtungen für die Beimischung erforderlich.

Bei den Zeolithen handelt es sich um Gerüstsilikate, in denen in Polyedern, Schichten oder Ketten eckverknüpfte [(AlSi)O₄]-Tetraeder vorliegen, die zu einem porenreichen, von langen Kanälen durchzogenen, anionischen Raumnetzwerk verbunden sind. Eine Verwendungsmöglichkeit der Zeolithe ist die als Molekularsiebe. Die hier verwendeten Zeolithe sollen nicht vom fasrigen Typ sein, da diese ähnlich wie Asbest Sicherheitsrisiken bergen. Darüber hinaus sind die hier bevorzugten Zeolithe im wesentlichen hydrophob, da sie in aller Regel Gerüche im Beisein von Körperflüssigkeiten absorbieren sollen.

Die Geruchsadsorptionsfähigkeit dieser als Molekularsiebe ausgebildeten Zeolithe wird durch Abfangen der schlecht riechenden Substanzen durch chemische Adsorption innerhalb ihrer molekularen Strukturen gebildet. Es ist dabei wichtig, daß die inneren Öffnungen oder Käfige der Zeolithmoleküle offen und frei sind für die geruchsverursachenden Moleküle.

Durch die Bindung der Zeolithe, die generell eine sehr geringe Partikelgröße aufweisen, an größere Partikel, nämlich ein thermoplastisches Granulat, das in seiner Partikelgröße problemlos eingestellt werden kann, lassen sich die üblichen Handhabungsprobleme der Zeolithe, wie Staubentwicklung und - entmischung, sicher verhindern.

Durch die Bindung der Zeolithe über Schmelzbindungen an das thermoplastische Granulat wird zum einen eine sichere Verankerung der Zeolithe an dem Granulat sichergestellt, es erfolgt jedoch keine chemische Veränderung weder des Granulats noch der Zeolithe, so daß die Adsorptionseigenschaften der Zeolithe voll erhalten bleiben.

Darüber hinaus wird die Oberfläche der Zeolithe durch die Schmelzklebeverbindung nur geringfügig verringert.

Die Verbindung läßt sich schließlich auf einfache technische Weise realisieren.

Als thermoplastisches Granulat kommt insbesondere Polypropylen, Polyäthylen, Polyamid oder EVA in Frage.

Die Zeolithe sind vorzugsweise kleiner und insbesondere sehr viel kleiner als die Partikel des thermoplastischen Granulats, an dem sie über Schmelzbindungen angeheftet sind. So können sehr viele Zeolithpartikel an einem einzigen Granulatpartikel gebunden sein. Insbesondere kann die Größe des Granulates das 10-fache, und insbesondere das 100-fache und insbesondere das mehr als 100-fache der Größe der Zeolithpartikel betragen.

Das Granulat kann eine Partikelgröße von 50 bis 2000 µm, insbesondere von 100 bis 1000 µm und insbesondere von 200 bis 800 µm aufweisen. Im Gegenzug besitzen die Zeolithe vorzugsweise eine Partikelgröße von 0,1 bis 50 µm und insbesondere von 1 bis 10 µm. In diesem Größenbereich ist eine besonders gute effektive Geruchsadsorption insbesondere für die geruchsbildenden Moleküle von Körperflüssigkeiten gegeben.

Im Bereich des Einsatzes für Hygieneartikel ist insbesondere die Kombination eines thermoplastischen Granulats mit einer Partikelgröße von 200 bis 800 µm mit Zeolithen mit einer Partikelgröße von 1 bis 10 µm geeignet. Diese Verbindung bietet Vorteile bezogen auf die Trägeroberfläche, die Kosten und die Geruchsadsorption bezogen auf einen effektiven Zeolitheinsatz sowie die Zudosierbarkeit in Hygieneartikel.

Als Zeolithe eignen sich z. B. das Zeolith der Firma UOP, das unter dem Namen Abscents 3000 vertrieben wird bzw. das Zeolith der Firma Degussa, das den Namenmarken Flavith S108 trägt.

Die Erfindung betrifft des weiteren einen Hygieneartikel, insbesondere eine Damenbinde, eine Windel oder einen Tampon, umfassend ein vorstehend beschriebenes Geruchsadsorptionsmittel.

Gerade in Hygieneartikeln ist die Verwendung eines Geruchsadsorptionsmittels empfehlenswert und erhöht den Tragekomfort der genannten Hygieneartikel. Viele der austretenden Körperflüssigkeiten weisen zumindest im Zusammentritt mit Luft und Bakterien eine unangenehme Geruchsbildung auf, die durch Zudosierung und Verwendung von Geruchsadsorbentien eingedämmt oder verhindert werden kann.

Es kann dabei vorgesehen sein, daß der Hygieneartikel eine absorbierende Struktur umfaßt, die ein superabsorbierendes Polymer als Absorptionsmittel für die aufzunehmende Körperflüssigkeit aufweist. Derartige superabsorbierende Polymere (SAP) werden vielfach wegen ihrer hervorragenden Speicher- und Bindungseigenschaften für Flüssigkeiten in Hygieneartikeln verwendet. Bei SAPs handelt es sich um gelbildende Polymere, die bei Zutritt von Wasser von ihrer ursprünglichen Pulverform durch Aufquellen in eine gelige Form überführt werden.

Dabei kann ein Vielfaches an Flüssigkeit sicher und schnell gebunden werden. Die Gefahr des Rewetting wird deutlich gesenkt.

Es kann dabei vorgesehen sein, daß das superabsorbierende Polymer in Granulatform vorliegt. Das superabsorbierende Polymer und das Geruchsadsorptionsmittel können dabei als Gemisch in dem Hygieneartikel vorhanden sein. Dies bietet den Vorteil, daß diese zusammen dem absorbierenden Kern zugeführt und zudosiert werden können. Weitere Dosiereinrichtungen sind dann nicht erforderlich.

Es kann jedoch auch vorgesehen sein, daß eine separate Zudosierung erfolgt. Auf diese Weise können besonders einfach Rezepturen mit unterschiedlicher Geruchsadsorptionscharakteristik hergestellt werden. Zur Zudosierung eignen sich Aggregate, wie sie aus der SAP-Dosierung bekannt und üblich sind. Es kann insbesondere vorgesehen sein, daß das superabsorbierende Polymer und das Geruchsadsorptionsmittel geschichtet in dem Hygieneartikel angeordnet sind.

Besonders günstige Eigenschaften hinsichtlich des Mischungsverhaltens ergeben sich, wenn die Partikelgröße des SAP und die des Geruchsadsorptionsmittels im wesentlichen gleich sind.

Des weiteren betrifft die Erfindung ein Verfahren zur Herstellung eines Geruchsadsorptionsmittels, wie es vorstehend beschrieben ist. Das thermoplastische Granulat wird dabei als Träger in einem beheizbaren Mischer oberflächlich angeschmolzen und die Zeolithe werden dem Granulat zudosiert. Sie verkleben dabei mit der angeschmolzenen Hülle des Granulats über Schmelzbindungen.

Die Mischbewegung kann beim Abkühlen fortdauern, bis die Hülle des Granulats im wesentlichen erstarrt ist, um ein Verkleben der Granulatteilchen untereinander zu vermeiden. Das Anschmelzen erfolgt dabei nur oberflächlich, wobei der Kern des Granulats fest bleibt. Die Zeolithe verkleben mit der angeschmolzenen Oberfläche, ohne zu tief in das Granulat einzudringen und dadurch ihre Geruchsadsorptionsfähigkeit zu verlieren.

Beim Absenken der Temperatur soll die Misch- bzw. Rührbewegung weiter andauern, denn insbesondere bei nur geringer Zeolithbeladung kann es sonst zu einem Verkleben der Granulatteilchen untereinander kommen.

Entsprechende Mischer sind aus dem Stand der Technik bekannt und können kontinuierlich oder diskontinuierlich ausgeführt sein.

Die Erfindung soll im folgenden anhand eines Beispieles näher erläutert werden, dabei zeigen:

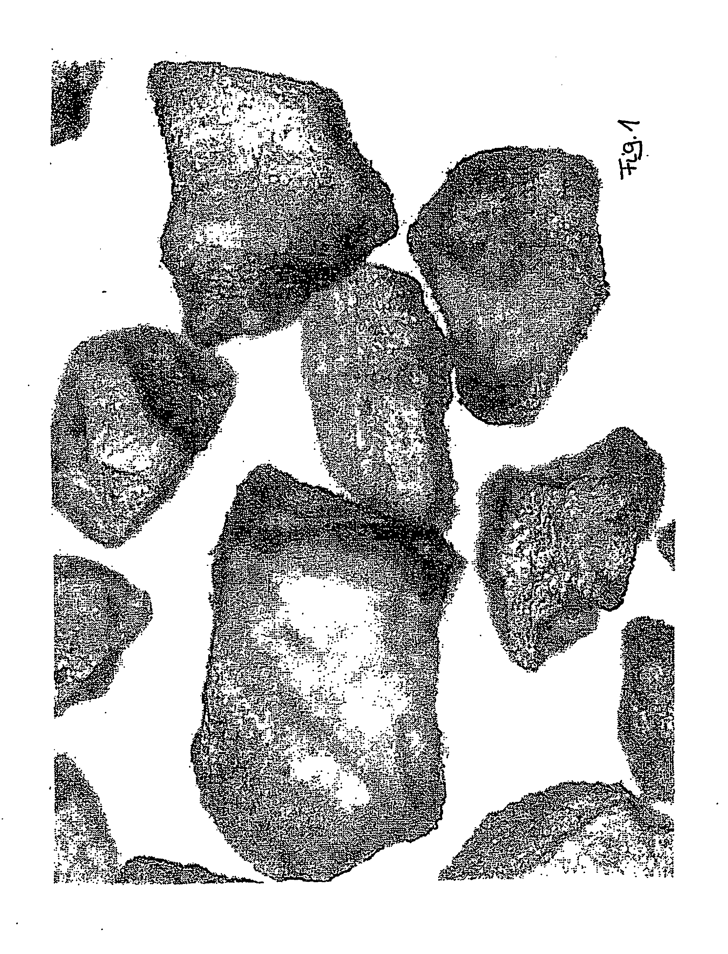

- Figur 1: verschiedene Granulatpartikel und
- Figur 2: ein Granulatpartikel in vergrößerter Darstellung mit Zeolith belegt.

Im Beispiel wurde als Thermoplast ein Co-Polyamid-Granulat, das als Schmelzkleber für die Textilindustrie dient, mit einer Korngröße zwischen 200 bis 500 µm und einem Schmelzpunkt von 105° C sowie einem Haftpunkt von ca. 70° C ausgewählt.

Mit Hilfe eines speziellen Mischers, der als beheizbarer Schermischer diskontinuierlich ausgestaltet ist, wurden 10 % Zeolith auf der Oberfläche eines geeigneten Trägermaterials aufgebracht. Aufgetragen wurde als Zeolith Abscents 3000 mit einem Partikeldurchmesser von ca. 3 µm und Flavit S108 mit einem Partikeldurchmesser von ca. 10 µm. Bei dem Zeolith Abscents 3000 handelt es sich um ein Zeolith der Firma UOP und bei Flavith S108 um ein Zeolith der Firma Degussa.

Nach einer ausreichenden Aufheizphase wurden die Produkte in einem nachgeschalteten Mischer abgekühlt und anschließend entnommen.

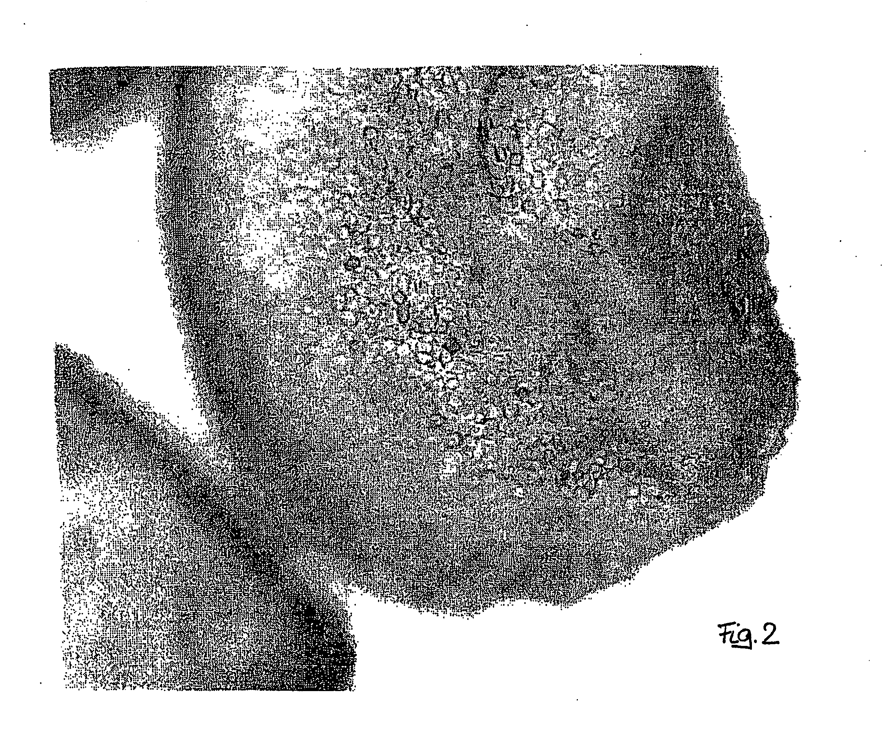

Die Produkte weisen nach ihrer Entnahme aus dem Mischer augenscheinlich keinen Feinstoff mehr auf, d. h. die Zeolithe scheinen komplett durch das Granulat aufgezogen. Mikroskopische Aufnahmen der Produkte bestätigen diesen Eindruck, wie auf der beigefügten Figur 2 gut zu erkennen ist. Auf ihr ist die dichte Belegung der Oberfläche der Co-Polyamid-Granulate mit den Zeolithpartikeln zu erkennen.

Es ergeben sich dabei folgende Kenndaten:
Flavith S108-Typ (Produkt A):
   SiO₂-Gehalt 6,9 %, Schüttdichte 545 g/l,
Abscents 3000-Typ (Produkt B):
   SiO₂-Gehalt 7,3 %, Schüttdichte 530 g/l.

Zur Kontrolle des Geruchsbindevermögens von Gemischen aus einem SAP I und mit den Zeolithen beschichteten Thermoplasten wurden unterschiedliche Abmischungen hergestellt. Die Geruchsbindung bei Fufurylmercaptan als Testsubstanz, beim Zusatz von 5 bis 10 % Geruchsbinder (Produkt A oder B) zu dem SAP I war im Durchschnitt sehr gut. Vergleicht man Produkte mit 0,5 % Abscents 3000 auf einem SAP II mit Abmischungen SAP I plus 5 % Produkt B, so ist das Geruchsbindevermögen vergleichbar. Von den Einwaagemengen enthalten beide Produkte den gleichen Zeolith-Anteil.

Im Laborversuch ließen sich Proben SAP I plus Produkt A oder B durch Förderung oder mechanische Vibration nicht entmischen. Ebenso fielen bei der Dosierung der Abmischungen in der Airlaid-Apparatur keine Inhomogenitäten in den gefertigten Airlaids auf.

Die aus 5 und 10 % Produkt B plus SAP I hergestellten Abmischungen zeigten in den gefertigten Airlaids hinsichtlich der Einsickerzeit und des Rewettingverhaltens mit SAP I vergleichbare Ergebnisse.

Weitere Merkmale der Erfindung ergeben sich aus den übrigen Anmeldungsunterlagen.

## Patentansprüche

1. Geruchsadsorptionsmittel, insbesondere für die Verwendung in Hygieneartikeln, umfassend ein thermoplastisches Granulat sowie über Schmelzbindungen an das thermoplastische Granulat gebundene Zeolithe.

2. Geruchsadsorptionsmittel nach Anspruch 1, wobei das thermoplastische Granulat aus Polypropylen, Polyamid, Polyethylen oder EVA (Ethylen/Vinylacetat-Copolymere) besteht.

3. Geruchsadsorptionsmittel nach Anspruch 1 oder 2, wobei das thermoplastische Granulat eine Partikelgröße von 50 bis 2000 µm, inbesondere von 100 bis 1000 µm und insbesondere von 200 bis 800 µm aufweist.

4. Geruchsadsorptionsmittel nach einem oder mehreren der Ansprüche 1 bis 3, wobei die Zeolithe pulverförmig sind und eine Partikelgröße von 0,1 bis 50 µm, insbesondere von 1 bis 10 µm aufweisen.

5. Hygieneartikel, insbesondere eine Damenbinde, Windel oder Tampon, umfassend ein Geruchsadsorptionsmittel nach einem oder mehreren der vorstehenden Ansprüche.

6. Hygieneartikel nach Anspruch 5, wobei der Hygieneartikel eine absorbierende Struktur umfaßt, die ein superabsorbierendes Polymer (SAP) als Absorptionsmittel für die aufzunehmenden Körperflüssigkeiten aufweist.

7. Hygieneartikel nach Anspruch 6, wobei das superabsorbierende Polymer in Granulatform vorliegt und das superabsorbierende Polymer und das Geruchsadsorptionsmittel als Gemisch in dem Hygieneartikel vorliegen.

8. Hygieneartikel nach Anspruch 6, wobei das superabsorbierende Polymer und das Geruchsadsorptionsmittel geschichtet in dem Hygieneartikel angeordnet sind.

9. Hygieneartikel nach einem oder mehreren der Ansprüche 5 bis 8, wobei die Partikelgröße des SAP und des Geruchsadsorptionsmittels im wesentlichen gleich sind.

10. Verfahren zur Herstellung eines Geruchsadsorptionsmittels nach einem oder mehreren der Ansprüche 1 bis 4, wobei das thermoplastische Granulat in einem beheizbaren Mischer oberflächlich angeschmolzen und die Zeolithe dem Granulat zudosiert werden und mit der angeschmolzenen Hülle der Granulatteilchen verkleben.

11. Verfahren zur Herstellung eines Geruchsadsorptionsmittels nach Anspruch 10, wobei bei der Abkühlung die Mischbewegung fortdauert, bis die Oberfläche der Granulatteilchen im wesentlichen erstarrt ist, um ein Verkleben der Granulatteilchen untereinander zu vermeiden.

## Claims

1. Odour adsorption means, in particular for use in hygiene products, comprising a thermoplastic granular material and also zeolites bound to the thermoplastic granular material via fusion bonds.

2. Odour adsorption means according to claim 1, wherein the thermoplastic granular material consists of polypropylene, polyamide, polyethylene or EVA (ethylene/vinyl acetate copolymer).

3. Odour adsorption means according to either of claims 1 and 2, wherein the thermoplastic granular material has a particle size of 50 to 2000 µm, in particular of 100 to 1000 µm, and more particularly of 200 to 800 µm.

4. Odour adsorption means according to one or more of claims 1 to 3, wherein the zeolites are pulverulent and having a particle size of 0.1 to 50 µm, in particular of 1 to 10 µm.

5. Hygiene product, in particular a sanitary towel, nappy or tampon, comprising an odour adsorption means according to one or more of the preceding claims.

6. Hygiene product according to claim 5, wherein the hygiene product comprises an absorbent structure, which comprises a superabsorbent polymer (SAP) as an absorption means for the body fluids to be absorbed.

7. Hygiene product according to claim 6, wherein the superabsorbent polymer is present in granular form, and the superabsorbent polymer and the odour adsorption means is present as a mixture in the hygiene product.

8. Hygiene product according to claim 6, wherein the superabsorbent polymer and the odour adsorption means are arranged in the hygiene product in a stratified manner.

9. Hygiene product according to one or more of claims 5 to 8, wherein the particle sizes of the SAP and of the odour adsorption means are substantially equal.

10. Method for producing an odour adsorption means according to one or more of claims 1 to 4, wherein the thermoplastic granular material is superficially melted in a heatable mixer, and the zeolites are added to the granular material and adhere to the melted casing of the granular material particles.

11. Method for producing an odour adsorption means according to claim 10, wherein the mixing movement continues during cooling until the surface of the granular material particles has substantially solidified, in order to prevent the granular material particles from adhering to one another.

## Revendications

1. Agent d'absorption, destiné à être utilisé en particulier dans des articles d'hygiène, comprenant un granulat thermoplastique, ainsi que des zéolithes liées au granulat thermoplastique par des liaisons de fusion.

2. Agent d'absorption selon la revendication 1, dans lequel le granulat thermoplastique est en polypropylène, en polyamide, en polyéthylène ou en EVA (copolymère éthylène/acétate de vinyle).

3. Agent d'absorption selon la revendication 1 ou 2, dans lequel le granulat thermoplastique présente une granulométrie comprise entre 50 et 2000 µm, en particulier entre 100 et 1000 µm et en particulier entre 200 et 800 µm.

4. Agent d'absorption selon l'une ou plusieurs des revendications 1 à 3, dans lequel les zéolithes sont pulvérulentes et présentent une granulométrie comprise entre 0,1 et 50 µm, en particulier entre 1 et 10 µm.

5. Article d'hygiène, en particulier une garniture féminine, une couche ou un tampon, comprenant un agent d'absorption d'odeur selon l'une ou plusieurs des revendications précédentes.

6. Article d'hygiène selon la revendication 5, comprenant une structure absorbante qui présente un polymère superabsorbant (SAP) en tant qu'agent d'absorption pour les liquides corporels à absorber.

7. Article d'hygiène selon la revendication 6, dans lequel le polymère superabsorbant est présent sous forme de granulat et le polymère superabsorbant et l'agent d'absorption d'odeur sont présents en tant que mélange dans l'article d'hygiène.

8. Article d'hygiène selon la revendication 6, dans lequel le polymère superabsorbant et l'agent d'absorption d'odeur sont disposés en couches dans l'article d'hygiène.

9. Article d'hygiène selon l'une ou plusieurs des revendications 5 à 8, dans lequel la granulométrie du polymère superabsorbant (SAP) et de l'agent d'absorption d'odeur est essentiellement identique.

10. Procédé pour fabriquer un agent d'absorption d'odeur selon l'une ou plusieurs des revendications 1 à 4, dans lequel le granulat thermoplastique est fondu superficiellement dans un mélangeur pouvant être chauffé et les zéolithes sont dosées et additionnées au granulat, puis collées à l'enveloppe fondue des particules de granulat.

11. Procédé pour fabriquer un agent d'absorption d'odeur selon la revendication 10, dans lequel le mouvement de mélange continue, lors du refroidissement, jusqu'à ce que la surface des particules de granulat soit essentiellement solidifiée, afin d'éviter le collage des particules de granulat entre elles.
